# EUROPEAN PATENT APPLICATION

(11) **EP 2 853 218 A1**
(43) Date of publication of application: **01.04.2015**
(21) Application number: 14186520.4
(22) Date of filing: 26.09.2014
(51) Int. Cl.: A61B 18/12, B65H 75/28, B65H 75/36

(54) **Cable management system for medical treatment device**

(30) Priority: 29.09.2013 WO PCT/CN2013/084576
(71) Applicant: Covidien LP, Mansfield, MA 02048 (US)
(72) Inventor: Wang, Li, Shanghai 201114 (CN); Zhang, Quan, Shanghai 201114 (CN); Hong, Chnunlang, Shanghai 201114 (CN)
(74) Representative: Gray, James

(57) **Abstract**

An enclosure of a power source for medical treatment devices includes a handle portion extending from a first surface of the enclosure. The handle portion includes a standoff extending away from the first surface, and a flange extending transversely from the standoff and defining a plane that is generally parallel to the first surface. Together, the first surface, the standoff, and the flange define an open annular space. A power cable may be wound about the standoff to at least partially occupy the open annular space. One or more notches in the flange are configured to seat the power cable to secure its loose end(s). In this manner, the power cable can be safely stored to reduce the risks that the power cable may become damaged, may interfere with other devices in the treatment area, or may be misplaced.

## Description

### Technical Field

The present embodiments relate to medical treatment devices, and in particular to a cable management system for a medical treatment device.

### Background

Various medical procedures use a power source that includes a cable and handle that connect to a medical treatment device, such as radio frequency ablation devices and radio frequency (RF) generators that drive heating segments. For example, one endovenous treatment for venous reflux disease, as well as other diseases in a hollow anatomical structure (HAS), is to apply energy from an RF generator to a heating segment within the HAS. The heating segment uses radio frequency heating to create targeted tissue ablation to seal off damaged veins. The RF equipment typically includes an RF generator and a catheter having a heating segment located at the distal end, which is inserted into the vein(s) during treatment. The heating segment uses RF energy driven by the RF generator to heat and seal the vein.

A power cable generally extends between the RF generator and the catheter. Typically, the generator remains outside the sterile field, and the power cable is long enough to reach into the sterile field. The cable must also be long enough to enable the user to easily maneuver and manipulate the device. When not in use, the power cable should be arranged and kept in an orderly fashion to, for example, prevent misuse, contamination, damage, loss, or interference with other components and/or users.

### SUMMARY

The present invention provides a system to manage a power cable and handle or medical treatment device such that the power cable and handle are stored on the power source, yet easily accessible. The various embodiments of the present cable management system have several features. Without limiting the scope of the present embodiments as expressed by the claims that follow, their features now will be discussed briefly. After considering this discussion, and particularly after reading the section entitled "Detailed Description," one will understand how the features of the present embodiments provide the advantages described herein.

In general, in one aspect, the implementation of the disclosure features a medical treatment system including a power source having an enclosure with a first surface. An enclosure handle portion including a standoff extends from the first surface of the enclosure, and a flange extends from the standoff. The flange defines a plane that is substantially parallel to the enclosure first surface, and the standoff, the flange, and the enclosure first surface define boundaries of an open annular space. The medical treatment system also includes an elongate power cable having a proximal end configured for coupling to the power source, and a distal end. A handle is disposed at the distal end of the power cable, and the handle includes a body portion and a distal end configured for coupling to a treatment apparatus. The flange includes a depression having a size and shape configured for matingly receiving the handle. The system further includes a first magnetic portion within the depression, and a second magnetic portion within the handle. The first and second magnetic portions are configured to retain the handle within the depression via a magnetic attraction between the first and second magnetic portions.

One or more of the following features may be included. The flange may define an outer edge having at least one notch. The at least one notch may include an inner portion spaced from the flange outer edge and have a diameter configured for receiving the power cable. The at least one notch may further include an outer portion adjacent the flange outer edge, and have a minimum width smaller than a diameter of the power cable.

The annular space is configured for receiving the power cable such that the power cable is wound around the standoff.

The first magnetic portion and the second magnetic portion may be selected from the group comprising a magnet and a ferromagnetic material. Alternatively, the first magnetic portion may be one of a pair of first magnets, a pair of first ferromagnetic materials, and a combination of a first magnet and a first ferromagnetic material. Similarly, the second magnetic portion may be one of a pair of second magnets, a pair of second ferromagnetic materials, and a combination of a second magnet and a second ferromagnetic material. The first magnetic portion may be recessed beneath an outer surface of the depression. Further, the first magnetic portion may be covered by the outer surface of the depression. The second magnetic portion may be recessed beneath an outer surface of the handle. The second magnetic portion may be covered by the outer surface of the handle.

In general, in another aspect, the implementation of the disclosure features a power source for a medical treatment system. The power source includes an enclosure having a first surface, and a handle portion including a standoff extending from the first surface of the enclosure with a flange extending from the standoff. The flange defines a plane that is substantially parallel to the enclosure first surface, and the standoff, the flange, and the enclosure first surface define boundaries of an open annular space. The flange also includes a depression with a magnetic portion within the depression.

One or more of the following features may be included. The annular space may be configured to receive an elongate power cable such that the power cable is wound around the standoff.

The flange defines an outer edge having at least one notch. The at least one notch includes an inner portion having a diameter spaced from the flange outer edge. The at least one notch may further include an outer portion adjacent the flange outer edge, such that the outer portion has a minimum width smaller than the diameter of the inner portion.

The magnetic portion may be selected from the group comprising a magnet and a ferromagnetic material. Alternatively, the magnetic portion may be selected from the group comprising a pair of magnets, a pair of ferromagnetic materials, and a combination of a magnet and a ferromagnetic material. The magnetic portion may be recessed beneath an outer surface of the depression. Further, the magnetic portion may be covered by the outer surface of the depression.

In general, in still another aspect, the implementation of the disclosure features a medical treatment system including a power source having an enclosure. The enclosure includes a first surface, and an enclosure handle portion including a standoff extending from the first surface of the enclosure with a flange extending from the standoff. The flange defines a plane that is substantially parallel to the enclosure first surface, such that the standoff, the flange, and the enclosure first surface define boundaries of an open annular space. The medical treatment system also includes an elongate power cable having a proximal end configured for coupling to the power source, and a distal end. A handle is disposed at the distal end of the power cable, and includes a body portion. The flange also includes a depression, and the depression has a size and shape configured for receiving the handle.

One or more of the following features may be included. The flange may define an outer edge having at least one notch. The at least one notch may include an inner portion spaced from the flange outer edge and have a diameter configured for receiving the power cable. The at least one notch may further include an outer portion adjacent the flange outer edge and have a minimum width smaller than a diameter of the power cable.

The annular space may be configured for receiving the power cable such that the power cable is wound around the standoff.

In general, in a further aspect, the implementation of the disclosure features a method of storing an elongate power cable of a medical treatment system, in which the system includes a power source having an enclosure. The method includes winding the power cable around an enclosure handle portion including a standoff extending from a first surface of the enclosure and a flange extending from the standoff, wherein the flange defines a plane that is substantially parallel to the enclosure first surface. The standoff, the flange, and the enclosure first surface define boundaries of an open annular space into which the cable is wound. A handle at a distal end of the power cable is seated within a depression in the flange, wherein the depression has a size and shape configured for receiving the handle. The handle is releasably retained within the depression via a magnetic attraction between a first magnetic portion within the depression and a second magnetic portion within the handle.

One or more of the following features may be included. The method may also include seating the power cable within a notch defined by the flange. The power cable may be received within an inner portion of the notch, the inner portion being spaced from an outer edge of the flange. The power cable may be drawn through an outer portion of the notch, the outer portion being adjacent the flange outer edge and having a minimum width smaller than a diameter of the power cable.

The first magnetic portion and the second magnetic portion may be selected from the group comprising a magnet and a ferromagnetic material. Further, the first magnetic portion may be selected from a group comprising a pair of first magnets, a pair of first ferromagnetic materials, and a combination of a first magnet and a first ferromagnetic material. Further still, the second magnetic portion may be selected from a group comprising a pair of second magnets, a pair of second ferromagnetic materials, and a combination of a second magnet and a second ferromagnetic material.

The first magnetic portion may be recessed beneath an outer surface of the depression. Also, the first magnetic portion may be covered by the outer surface of the depression. In other embodiments, the second magnetic portion may be recessed beneath an outer surface of the handle. The second magnetic portion may be covered by the outer surface of the handle.

The invention may be implemented to realize one or more of the following advantages. It is advantageous to provide for storage of power cables for medical treatment devices so that the cables are not misplaced, damaged, or contaminated, and so that the cables do not interfere with other apparatus and/or people in the vicinity of a medical treatment procedure. The present embodiments provide convenient and orderly storage for power cables by providing a storage location that is integrated into the medical device. The storage location provides an open space for receiving the cable in a wound configuration, with loose ends of the cable secured. The cable is thus less likely to be misplaced, damaged, or contaminated, or to interfere with other apparatus and/or people. Further, the magnets provide an easy and secure way to retain the handle, or a treatment device, at the distal end of the cable, thereby preventing the handle from hanging loose from the cable, or being forced into the annular space. By retaining the handle in a secure, but easily accessible location, the handle is less susceptible to damage or misuse. Also, because the magnets provide a quick and easy way to retain the handle, or the treatment device, a user is more likely to properly store the handle after each use.

### BRIEF DESCRIPTION OF THE DRAWINGS

The present embodiments now will be discussed in detail with an emphasis on highlighting the advantageous features. These embodiments are for illustrative purposes only. These drawings include the following figures, in which like numerals indicate like parts:
Figure 1 is an overview of a medical treatment system;
Figures 2 and 3 are side elevation views of an example procedure using the medical treatment system of Figure 1;
Figure 4 is a front perspective view of one embodiment of a power source having a cable management system for a medical treatment device;
Figure 5 is a left-side elevation view of the power source having the cable management system of Figure 4;
Figure 6 is a front elevation view of the power source having the cable management system of Figure 4; and
Figure 7 is a top perspective view of a handle portion of the cable management system of Figure 4.

### DETAILED DESCRIPTION

The following detailed description describes the present embodiments with reference to the drawings. In the drawings, reference numbers label elements of the present embodiments. These reference numbers are reproduced below in connection with the discussion of the corresponding drawing features.

Directional terms used herein, such as proximal, distal, upper, lower, clockwise, counterclockwise, etc., are used with reference to the configurations shown in the figures. For example, a component that is described as rotating clockwise when viewed from the perspectives shown in the figures may be said to rotate counterclockwise when viewed from the opposite perspective. Furthermore, the present embodiments may be modified by altering or reversing the positions or directions of movement of various components. Accordingly, directional terms used herein should not be interpreted as limiting.

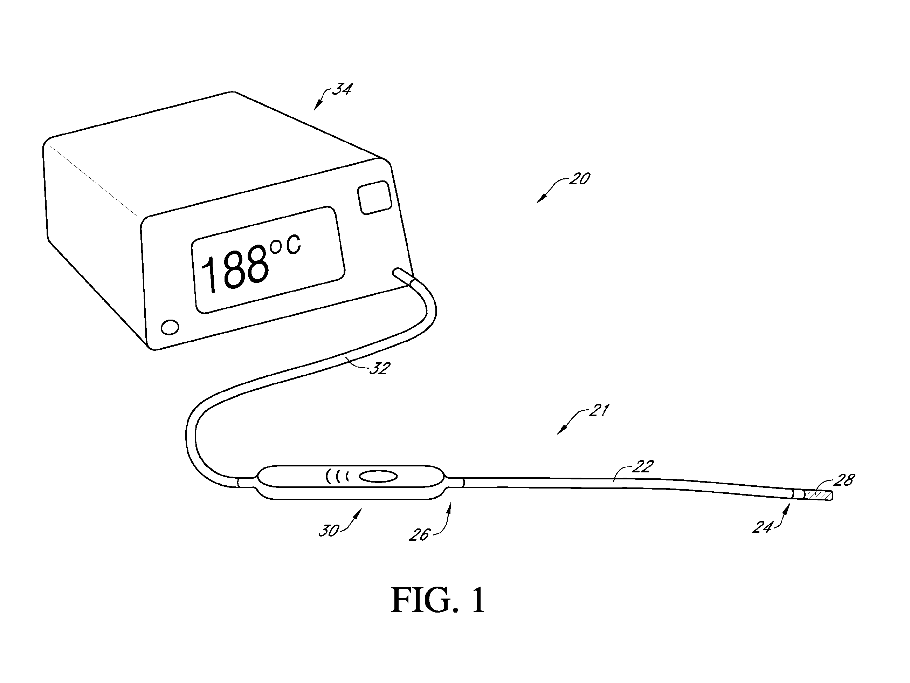

Referring to Figure 1, one example of a medical treatment system 20 may include a treatment apparatus 21 comprising a catheter shaft 22 having a distal end 24 and a proximal end 26. A heating segment 28 is operably attached adjacent the distal end 24 of the catheter shaft 22 and a handle 30 is attached at the proximal end 26 of the catheter shaft 22. The handle 30 may be integrally attached to the treatment apparatus 21, or, alternatively, the handle 30 may be removably attached to the treatment apparatus 21. A power cable 32 electrically connects the heating segment 28 to a power source 34. The power cable 32 may be integral to the handle 30 and removably connected to the power source 34. Alternatively, the power cable 32 may be removably connected to the handle 30. Alternatively, the handle 30 may comprise two sections having a first coupler portion and a second coupler portion, where the first and second coupler portions are attached to and/or integral with the power cable 32 and the treatment apparatus 21, respectively.

The heating segment 28 is secured at the distal end 24 of the elongate catheter shaft 22. The catheter shaft 22 may be used to maneuver the heating segment 28 into a desired placement within a HAS. In certain embodiments, the power source 34 comprises an alternating current (AC) source, such as an RF generator. In other embodiments, the power source 34 comprises a direct current (DC) power source, such as, for example, a battery, a capacitor, or other power source such as would be used for microwave heating. The power source 34 may also incorporate a controller that, through the use of a processor, applies power based at least upon readings from a temperature sensor or sensors (e.g., a thermocouple, a thermistor, a resistance temperature device, an optical or infrared sensor, combinations of the same or the like) located in or adjacent to the heating segment 28. For example, the controller may heat the tissue of a HAS or the heating segment 28 to a set temperature. In an alternative embodiment, the user selects a constant power output of the power source 34. For example, the user may manually adjust the power output relative to a temperature display from a temperature sensor in the heating segment 28.

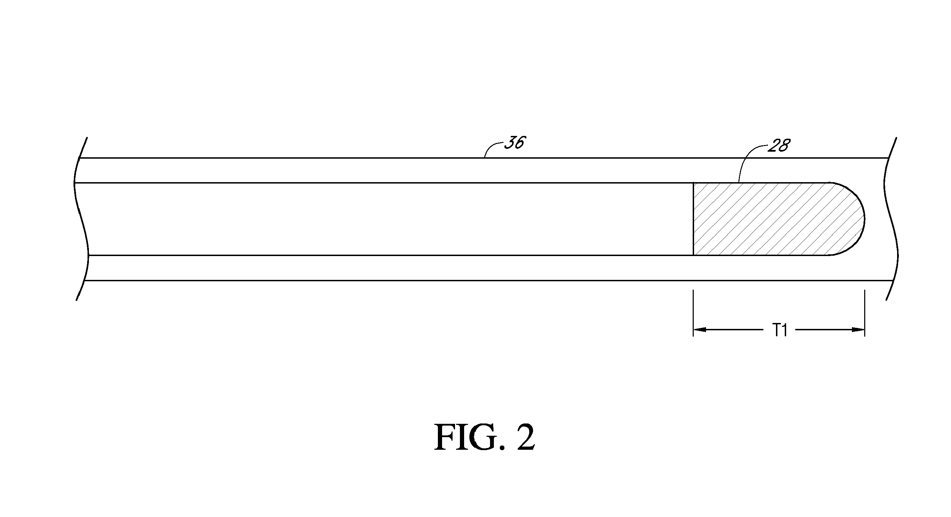

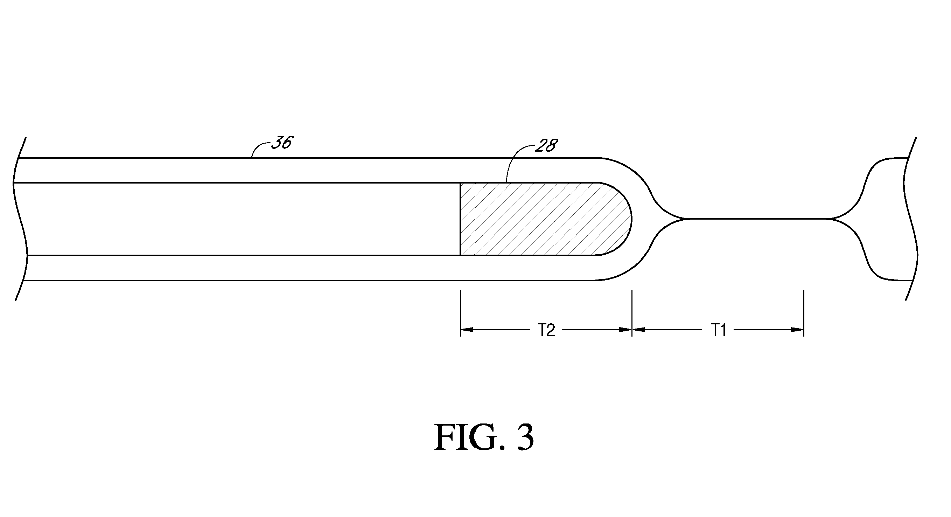

The medical treatment system 20 may be used in various medical procedures, including, for example, endovenous treatments to treat venous reflux. Specifically, referring to Figure 2, a method may comprise inserting the heating segment 28 into a distal-most section of a HAS 36 to be treated. The heating segment 28 is then aligned with a first treatment location T1 within the HAS 36. Power is then applied to the heating segment 28 for a desired length of time to treat the first treatment location T1. After a desired dwell time, such as after the HAS 36 has collapsed as shown in Figure 3, the power to the heating segment 28 may be reduced or shut off. With the power off (or substantially reduced), the heating segment 28 may then be moved proximally until the distal end of the heating segment 28 is adjacent to the proximal end of the first treatment location T1, as shown in Figure 3. At this second treatment location T2 within the HAS 36, power is again applied to the heating segment 28 for a desired length of time to treat the HAS 36 at the second treatment location T2. This process of withdrawing the heating segment 28 is repeated until the treatment of the HAS 36 is complete. In some embodiments, T1 and T2 may overlap.

The power source 34 is typically outside the sterile field, while the patient, and thus the treatment apparatus 21, are within the sterile field. The distance between the sterile field and the placement of the power source 34 outside the sterile field may require a power cable 32 of considerable length. After the procedure, the cable 32 and handle 30 should be neatly stored with the power source 34 for easy storage and quick setup for the next procedure.

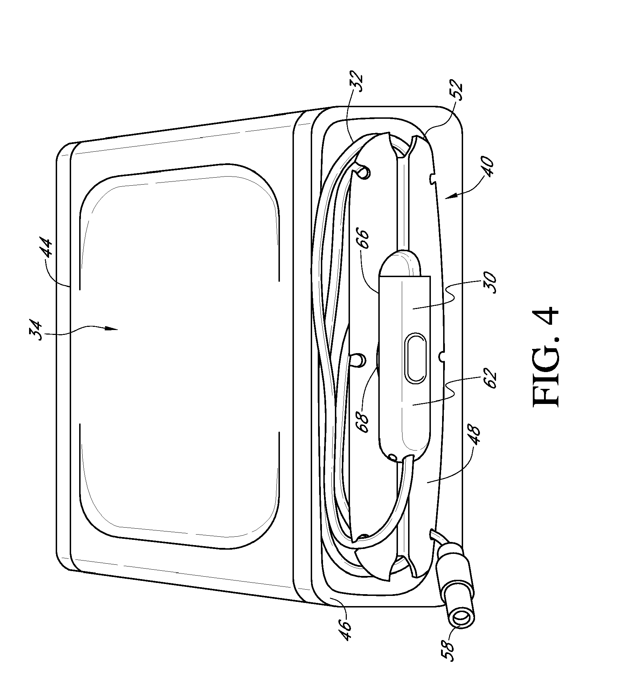

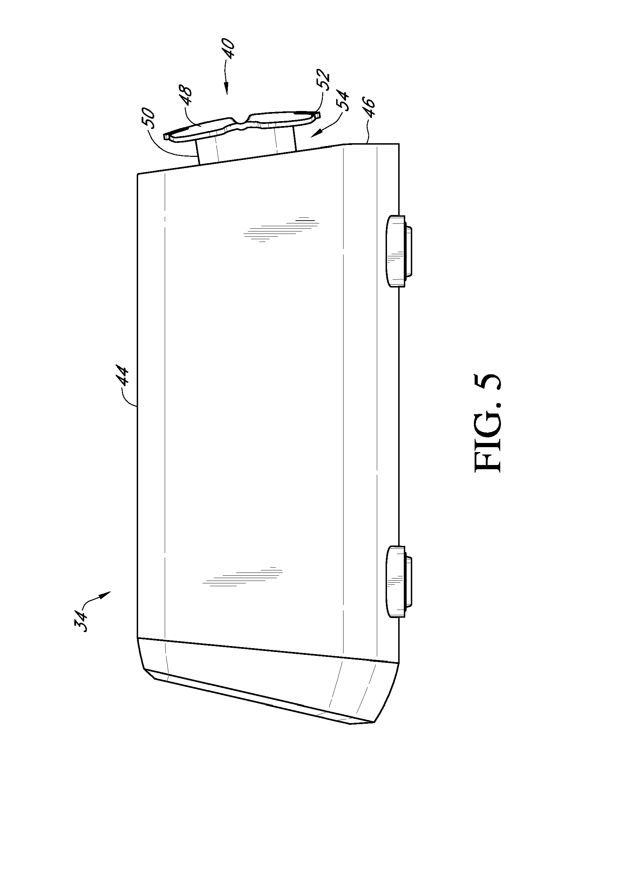

Figures 4-7 illustrate one embodiment of a cable management system 40 for use with the medical treatment system 20. With reference to Figures 4 and 5, the system 40 comprises a power source 34 including an enclosure 44 having a first surface 46. The power source 34 may include any internal components necessary for the power source 34 to function for its intended purpose. For example, with an RF generator, the power source 34 may include a controller, an RF circuit board, a user interface, a battery source and an AC to DC converter.

With reference to Figures 4-6, the enclosure 44 further comprises a handle portion 48 including a standoff 50 (Figures 5-6) extending from the first surface 46 of the enclosure 44 and a flange 52 extending from the standoff 50. In the illustrated embodiment, the standoff 50 is shaped substantially as an oval in cross-section, and defines a first circumference. The flange 52 defines a plane that is substantially parallel to the enclosure first surface 46. In the illustrated embodiment, the flange 52 is shaped substantially as an oval in cross-section, and defines a second circumference that is greater than the first circumference. The flange 52 thus extends outward in a radial direction from the standoff 50. With reference to Figure 5, the standoff 50, the flange 52, and the enclosure first surface 46 define boundaries of an open annular space 54. The open annular space 54 is configured for receiving the power cable 32, as illustrated in Figure 4, and as described in further detail below.

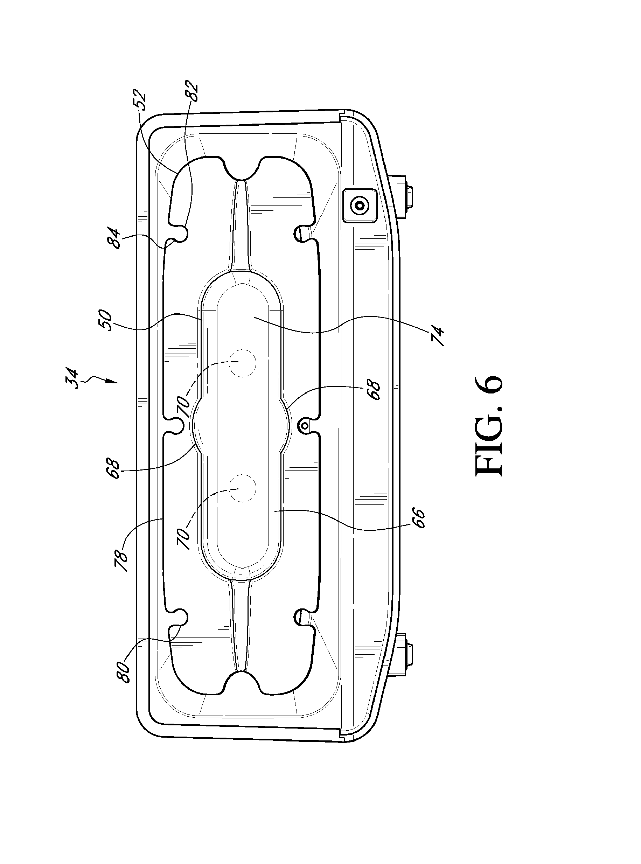

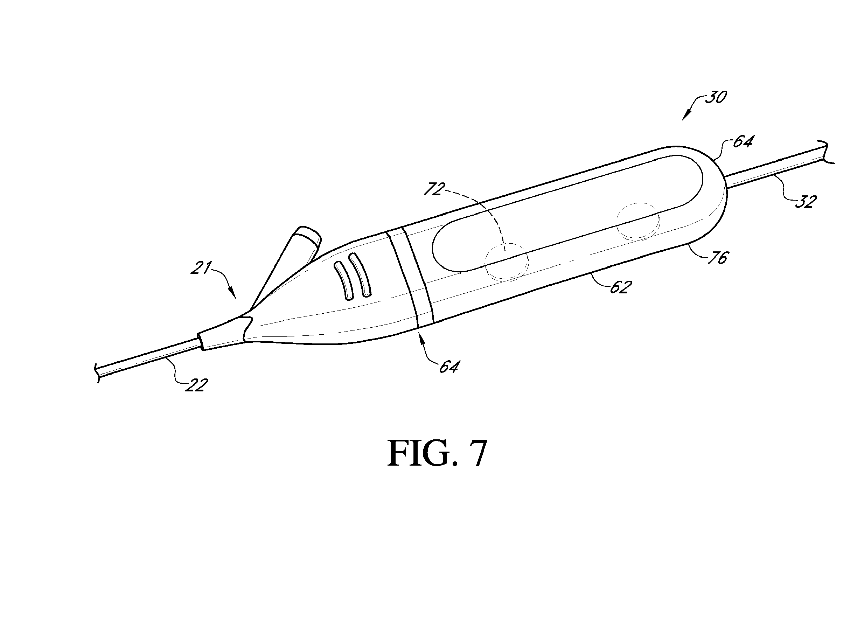

With reference to Figure 4, the cable management system 40 further comprises the elongate power cable 32. A proximal end of the power cable 32 may include a plug 58 configured for coupling with a socket (not shown) of the power source 34 to electrically connect the power cable 32 to the power source 34. Alternatively, the power cable 32 may be integrally coupled with the power source 34. The power cable 32 further includes a distal end coupled to the handle 30. With reference to Figure 7, the handle 30 includes a body portion 62 and a distal end 64 configured for coupling to a treatment apparatus, such as the treatment apparatus 21 shown in Figure 1, or any other type of treatment apparatus. With reference to Figure 6, a central portion of the flange 52 comprises a depression 66 having a size and shape configured for matingly receiving the handle 30, as shown in Figure 4. In the illustrated embodiment, the handle body portion 62 and the depression 66 are substantially oval shaped. A circumference of the depression 66 is slightly larger than a circumference of the handle body portion 62, and a depth of the depression 66 may be equal to about half of the thickness of the handle body portion 62. The depression 66 is thus configured to snugly seat the handle body portion 62 with a lower half of the handle body portion 62 seated within the depression 66 and an upper half of the handle body portion 62 extending outwardly of the depression 66, as shown in Figure 4. Opposite edges of the depression 66 include arcuate recesses 68 that provide space for an operator's fingers to grasp the handle 30 and pull it out of the depression 66.

With reference to Figure 6, the power source 34 further comprises a first magnetic portion 70 within the depression 66. With reference to Figure 7, the handle 30 further comprises a second magnetic portion 72 within the body portion 62. The first and second magnetic portions 70, 72 are configured to retain the handle 30 within the depression 66 via a magnetic attraction between the first and second magnetic portions 70, 72. The first and second magnetic portions 70, 72 may comprise, for example, magnets and/or ferromagnetic materials. For example, the first magnetic portion 70 may be selected from a group comprising a pair of first magnets, a pair of first ferromagnetic materials, and a combination of a first magnet and a first ferromagnetic material, and the second magnetic portion 72 may be selected from a group comprising a pair of second magnets, a pair of second ferromagnetic materials, and a combination of a second magnet and a second ferromagnetic material. In the illustrated embodiment, each of the first and second magnetic portions 70, 72 includes two spaced magnets and/or ferromagnetic materials. In alternative embodiments, any number of magnets and/or ferromagnetic materials may be provided for either or both of the first and second magnetic portions 70, 72.

With reference to Figure 6, the first magnetic portion 70 is recessed beneath an outer surface 74 of the depression 66, and covered by the outer surface 74 of the depression 66. With reference to Figure 7, the second magnetic portion 72 is recessed beneath an outer surface 76 of the handle 30, and covered by the outer surface 76 of the handle 30. In alternative embodiments, either or both of the first and second magnetic portions 70, 72 may be recessed beneath, but exposed from, its respective outer surface. In still further alternative embodiments, either or both of the first and second magnetic portions 70, 72 may be secured to its respective outer surface and not recessed beneath its respective outer surface.

With reference to Figures 4 and 5, the open annular space 54 defined by the standoff 50, the flange 52, and the enclosure first surface 46 is configured for receiving the power cable 32 such that the power cable 32 is wound around the standoff 50. The open annular space 54 thus advantageously provides an orderly storage location for the power cable 32, thereby reducing the likelihood that the power cable 32 will interfere with other components and/or users, or will be misused, contaminated, damaged, lost, etc. In the illustrated embodiment, the plug 58 is disconnected from the socket of the power source 34 when the power cable 32 is wound around the standoff 50, and the end of the power cable 32 adjacent the plug 58 is seated within one of the notches 80. In alternative embodiments, the plug 58 may remain connected to the socket of the power source 34 when the power cable 32 is wound around the standoff 50.

With reference to Figure 6, the flange 52 defines an outer edge 78 having a plurality of notches 80. In the illustrated embodiment, one notch 80 is provided adjacent each of the four locations of the flange 52 where the arcuate end portions meet the straight side edge portions, and along the top and bottom straight side edges, but the notches 80 may be located at any other location around the outer edge 78. Each of the notches 80 includes an inner portion 82 spaced from the flange 52 outer edge 78 and having a diameter configured for receiving the power cable 32, as shown in Figure 4. Each of the notches 80 further includes an outer portion 84 adjacent the flange 52 outer edge 78. The outer portion 84 has a minimum width that is smaller than a diameter of the power cable 32. When inserted into any of the notches 80, the power cable 32 resiliently deforms inwardly in order to squeeze through the relatively narrow outer portion 84. The power cable 32 then resiliently returns to its unstressed shape as it enters the inner portion 82. The smaller diameter of the outer portion 84 retains the power cable 32 within the notch 80 until a sufficient pulling force is applied to pull the power cable 32 through the outer portion 84 and out of the notch 80.

Certain of the present embodiments comprise a method of storing an elongate power cable of a medical treatment system. The system includes a power source having an enclosure. The enclosure comprises an enclosure handle portion including a standoff extending from a first surface of the enclosure and a flange extending from the standoff. The flange defines a plane that is substantially parallel to the enclosure first surface. The standoff, the flange, and the enclosure first surface define boundaries of an open annular space.

The method comprises winding the power cable around the standoff, seating a handle at a distal end of the power cable within a depression in the flange, the depression having a size and shape configured for receiving the handle, and releasably retaining the handle within the depression via a magnetic attraction between a first magnetic portion within the depression and a second magnetic portion within the handle.

The method may further comprise seating the power cable within a notch defined by the flange. The power cable may be received within an inner portion of the notch, the inner portion being spaced from an outer edge of the flange. The method may further comprise drawing the power cable through an outer portion of the notch, the outer portion being adjacent the flange outer edge and having a minimum width smaller than a diameter of the power cable.

It is to be understood that the foregoing description is intended to illustrate and not limit the scope of the invention, which is defined by the scope of the appended claims. Other embodiments are within the scope of the claims.

A power source for a medical treatment system, the power source comprising:
an enclosure having a first surface;
a handle portion including a standoff extending from the first surface of the enclosure and a flange extending from the standoff, the flange defining a plane that is substantially parallel to the enclosure first surface, the standoff, the flange, and the enclosure first surface defining boundaries of an open annular space;
a depression in the flange; and
a magnetic portion within the depression.

The power source described above, wherein the annular space is configured to receive an elongate power cable such that the power cable is wound around the standoff.

The power source described above, wherein the flange defines an outer edge having at least one notch.

The power source described above, wherein the at least one notch includes an inner portion spaced from the flange outer edge, the inner portion having a diameter.

The power source described above, wherein the at least one notch further includes an outer portion adjacent the flange outer edge, the outer portion having a minimum width smaller than the diameter of the inner portion.

The power source described above, wherein the magnetic portion is selected from the group comprising a magnet and a ferromagnetic material.

The power source described above, wherein the magnetic portion is selected from the group comprising a pair of magnets, a pair of ferromagnetic materials, and a combination of a magnet and a ferromagnetic material.

The power source described above, wherein the magnetic portion is recessed beneath an outer surface of the depression.

The power source of described above, wherein the magnetic portion is covered by the outer surface of the depression.

A medical treatment system, comprising:
a power source including an enclosure, the enclosure having a first surface;
an enclosure handle portion including a standoff extending from the first surface of the enclosure and a flange extending from the standoff, the flange defining a plane that is substantially parallel to the enclosure first surface, the standoff, the flange, and the enclosure first surface defining boundaries of an open annular space;
an elongate power cable having a proximal end configured for coupling to the power source, and a distal end;
a handle at the distal end of the power cable, the handle having a body portion; and
a depression in the flange, the depression having a size and shape configured for receiving the handle.

The system described above, wherein the flange defines an outer edge having at least one notch.

The system described above, wherein the at least one notch includes an inner portion spaced from the flange outer edge and having a diameter configured for receiving the power cable.

The system described above, wherein the at least one notch further includes an outer portion adjacent the flange outer edge and having a minimum width smaller than a diameter of the power cable.

The system described above, wherein the annular space is configured for receiving the power cable such that the power cable is wound around the standoff.

## Claims

1. A medical treatment system, comprising:
a power source including an enclosure, the enclosure having a first surface;
an enclosure handle portion including a standoff extending from the first surface of the enclosure and a flange extending from the standoff, the flange defining a plane that is substantially parallel to the enclosure first surface, the standoff, the flange, and the enclosure first surface defining boundaries of an open annular space;
an elongate power cable having a proximal end configured for coupling to the power source, and a distal end;
a handle at the distal end of the power cable, the handle having a body portion and a distal end configured for coupling to a treatment apparatus;
a depression in the flange, the depression having a size and shape configured for matingly receiving the handle;
a first magnetic portion within the depression; and
a second magnetic portion within the handle;
wherein the first and second magnetic portions are configured to retain the handle within the depression via a magnetic attraction between the first and second magnetic portions.

2. The system of Claim 1, wherein the flange defines an outer edge having at least one notch.

3. The system of Claim 2, wherein the at least one notch includes an inner portion spaced from the flange outer edge and having a diameter configured for receiving the power cable.

4. The system of Claim 3, wherein the at least one notch further includes an outer portion adjacent the flange outer edge and having a minimum width smaller than a diameter of the power cable.

5. The system of Claim 1, wherein the annular space is configured for receiving the power cable such that the power cable is wound around the standoff.

6. The system of Claim 1, wherein the first magnetic portion is recessed beneath an outer surface of the depression.

7. The system of Claim 6, wherein the first magnetic portion is covered by the outer surface of the depression.

8. The system of Claim 1, wherein the second magnetic portion is recessed beneath an outer surface of the handle.

9. The system of Claim 8, wherein the second magnetic portion is covered by the outer surface of the handle.

10. A method of storing an elongate power cable of a medical treatment system, the system including a power source having an enclosure, the method comprising:
winding the power cable around an enclosure handle portion including a standoff extending from a first surface of the enclosure and a flange extending from the standoff, the flange defining a plane that is substantially parallel to the enclosure first surface, the standoff, the flange, and the enclosure first surface defining boundaries of an open annular space;
seating a handle at a distal end of the power cable within a depression in the flange, the depression having a size and shape configured for receiving the handle; and
releasably retaining the handle within the depression via a magnetic attraction between a first magnetic portion within the depression and a second magnetic portion within the handle.

11. The method of Claim 10, further comprising seating the power cable within a notch defined by the flange.

12. The method of Claim 11, wherein the power cable is received within an inner portion of the notch, the inner portion being spaced from an outer edge of the flange.

13. The method of Claim 11, further comprising drawing the power cable through an outer portion of the notch, the outer portion being adjacent the flange outer edge and having a minimum width smaller than a diameter of the power cable.

14. The method of Claim 10, wherein the first magnetic portion is recessed beneath an outer surface of the depression.

15. The method of Claim 14, wherein the first magnetic portion is covered by the outer surface of the depression.

16. The method of Claim 10, wherein the second magnetic portion is recessed beneath an outer surface of the handle.

17. The method of Claim 16, wherein the second magnetic portion is covered by the outer surface of the handle.
